Europäisches Patentamt

European Patent Office

Office européen des brevets

(11) Veröffentlichungsnummer: **0 123 973**
A1

# (12) EUROPÄISCHE PATENTANMELDUNG

(21) Anmeldenummer: 84103997.7

(22) Anmeldetag: 10.04.84

(51) Int. Cl.³: **B 32 B 27/32,** B 65 D 65/40

(30) Priorität: 29.04.83 DE 3315652

(43) Veröffentlichungstag der Anmeldung: 07.11.84
Patentblatt 84/45

(84) Benannte Vertragsstaaten: **AT BE CH DE FR GB IT LI NL
SE**

(71) Anmelder: **Feldmühle Aktiengesellschaft,
Fritz-Vomfelde-Platz 4, D-4000 Düsseldorf 11 (DE)**

(72) Erfinder: **Engelsberger, Herbert, Heimbachstrasse 13,
D-4060 Viersen 1 (DE)**

(74) Vertreter: **Uhlmann, Hans, Dr. rer.nat., Dipl.-Chem.,
Gladbacher Strasse 189, D-4060 Viersen 1 (DE)**

(54) Mehrlagige, sterilisierbare Tiefziehfolie und daraus hergestellte Vakuumtiefziehpackung zur Aufnahme von Kunststoffbeuteln mit Blutinfusionslösung.

(57) Eine mehrlagige, sterilisierbare Tiefziehfolie, die als Unterfolie einer Vakuumtiefziehverpackung (2) mit darin verpacktem, Blutinfusionsflüssigkeit enthaltenden Kunststoffbeutel (5), zusammen mit einer an sich bekannten Oberfolie (3) verwendet wird, ist eine koextrudierte Verbundfolie (1') mit zwei in Material und Dicke übereinstimmenden Außenlagen (A, A') aus Polypropylen, einem Copolymeren des Propylens oder aus High-Density-Polyäthylen und zwei in Material und Dicke übereinstimmenden Innenlagen (B, B') eines Äthylen-Vinylacetatcopolymeren; die Außenlagen (A, A') sind mit den Innenlagen (B, B') durch Schmelzverbindung verbunden, während die Innenlagen (B, B') lediglich miteinander verblockt sind.

EP 0 123 973 A1

- 1 -

Mehrlagige, sterilisierbare Tiefziehfolie
und daraus hergestellte Vakuumtiefziehpackung
zur Aufnahme von Kunststoffbeuteln mit Blutinfusionslösung.

Die Erfindung betrifft mehrlagige, sterilisierbare
Tiefziehfolien, insbesondere für die Herstellung
vakuumtiefgezogener Verpackungen zur Aufnahme von in
Kunststoffbeuteln abgefüllten Blutinfusionsflüssigkeiten und eine unter Verwendung der Tiefziehfolien
zusammen mit einer Oberfolie hergestellten Vakuumtiefziehverpackung.

Mehrlagige, sterilisierbare Tiefziehfolien sind bekannt. Zur Verpackung von in Weich-PVC-Beuteln mit
Zapfventil abgefüllten Blutinfusionsflüssigkeiten
werden als Unterfolie tiefziehfähige Verbundfolien,
bestehend aus ungereckter Polyamid- oder Polyesterfolie, laminiert mit einer Folie aus High-Density-
Polyäthylen (HDPE, Niederdruckpolyäthylen) oder aus
Polypropylen ungereckt (PPUG) verwendet. Bei diesen
bekannten Folien besteht der Nachteil, daß zur Erzielung eines befriedigenden Tiefziehverhaltens eine
der beiden Folienlagen aus Polyamid oder Polyester
hergestellt werden muß und quasi als Stützfolie
während des Tiefziehvorganges fungiert. Man erreicht
bei Verwendung dieser relativ teuren Rohstoffklassen
zwar auch eine befriedigende Bruchfestigkeit, muß
aber in Kauf nehmen, daß die Verpackung insgesamt

- 2 -

wirtschaftlich nicht so günstig hergestellt werden kann, wie es in vielen Fällen verlangt wird.

Es sind auch Verfahren zur Herstellung von koextrudierten Verbundfolien bekanntgeworden, deren Anwendung die Herstellung einer zwei- oder mehrlagigen Folie in einem Arbeitsgang ermöglicht. Im wesentlichen werden nach diesen Verfahren Verbundfolien hergestellt, die in drei Gruppen unterteilt werden können:

Unter Verwendung von zwei oder mehr ringartig angeordneten Düsen hergestellte Schläuche, die

a) als solche zur Herstellung von Beutelverpackungen verwendet werden,
b) in gleicher Weise hergestellte Schlauchfolien, bei denen jedoch ein Auftrennen des Schlauches vorgenommen und die Folie als Flachfolie verwendet wird.

In beiden Fällen entstehen, z. B. bei Verwendung von zwei Ringdüsen, jeweils Folien mit zwei miteinander verbundenen Lagen. Solche Folien und Verfahren sind in der DE-AS 11 36 818 und in der DE-PS 12 89 297 beschrieben.

c) Koextrudierte Verbundschläuche, deren einzelne Lagen z. B. durch Versiegeln so zu einer einheitlichen Verbundfolie vereinigt sind, daß ein Ablösen der einzelnen Lagen voneinander nicht mehr möglich ist( DE-OS 19 66 466 und 21 02 377).

Auch diese mehrlagigen Folien haben das zur Lösung anstehende Problem, daß in der Entwicklung einer sterilisierbaren Tiefziehfolie mit hoher Knickbruchfestigkeit liegt, wobei die sterilisierbare Tiefziehfolie unter wirtschaftlichen Gesichtspunkten herzustellen ist, nicht lösen können.

Ein weiterer, technischer Nachteil der bekannten sterilisierbaren Tiefziehfolien ist die oft nicht ausreichende Knickbruchempfindlichkeit beim Evakuieren, verbunden mit einer gewissen Versprödung beim Erhitzen. Dadurch können beim Sterilisieren Knickbrüche auftreten, so daß zwischen Außen- und Innenverpackung, d. h. zwischen Unterfolie und Weich-PVC-Beutel, Reinfektionen entstehen können, wodurch der Weich-PVC-Beutel nicht mehr steril ist.

Ein weiterer Nachteil der bekannten Tiefziehfolien, der insbesondere bei komplizierteren Tiefziehformen auftritt, ergibt sich aus der hohen Sperrigkeit der Polyamid- oder Polyesterlagen. Infolge dieser Sperrigkeit ist eine formgenaue Ausbildung der Tiefziehfolie nicht immer möglich.

An diesem Punkte setzt die vorliegende Erfindung an, deren Aufgabe es ist, die bekannten Nachteile zu überwinden und eine sterilisierbare Tiefziehfolie mit verbesserter Knickbruchfestigkeit zur Verwendung als Unterfolie im Vakuumtiefziehverfahren, insbesondere für die Herstellung tiefgezogener Verpackungen zur Aufnahme von in Kunststoffbeuteln abgefüllten Blutinfusionsflüssigkeiten zur Verfügung zu stellen.

Die Erfindung hat auch die Aufgabe, eine sterilisierbare Tiefziehfolie (Unterfolie) für ein Vakuumtiefziehverfahren zur Verfügung zu stellen, die ein verbessertes Tiefziehverhalten aufweist und insbesondere eine formgenaue Ausbildung ermöglicht.

Desweiteren sieht die Erfindung ihre Aufgabe darin, die Herstellung einer sterilisierbaren Tiefziehfolie in einem einzigen Arbeitsgang, sowohl unter Einsatz kostengünstiger Rohstoffe als auch zu insgesamt günstigen Materialkosten zu ermöglichen.

Zur Lösung dieser Aufgabe sieht die Erfindung eine mehrlagige, sterilisierbare Tiefziehfolie, insbesondere für die Herstellung tiefgezogener Verpackungen zur Aufnahme von in Kunststoffbeuteln abgefüllten Blutinfusionsflüssigkeiten vor, die durch die folgenden Merkmale gekennzeichnet ist:
a) die mehrlagige Tiefziehfolie ist eine koextrudierte Verbundfolie (1') mit zwei in Material und Dicke übereinstimmenden Außenlagen (A,A') aus Polypropylen, einem Copolymeren des Polypropylens oder aus High-Density-Polyäthylen (Niederdruckpolyäthylen, HDPE) und zwei in Material und Dicke übereinstimmenden Innenlagen (B,B'), bestehend aus einem Äthylen-Vinylacetatcopolymeren,
b) die Außenlagen (A,A') sind mit den Innenlagen (B,B') durch Schmelzverbindung verbunden, die Innenlagen (B,B') sind lediglich miteinander verblockt.

Im Vergleich zu den bisher bekannten mehrlagigen Tiefziehfolien, die insbesondere für die Verpackung von in Kunststoffbeuteln abgefüllten Blut-

- 5 -

infusionsflüssigkeiten verwendet wurden, zeichnet sich die erfindungsgemäße Tiefziehfolie dadurch aus, daß sie aus im Vergleich zu Polyamid oder Polyester wesentlich kostengünstigeren Rohstoffen, wie aus Polypropylen, Copolymeren des Polypropylens oder High-Density-Polyäthylen bzw. aus Äthylen-Vinylacetatcopolymeren hergestellt ist. Ohne daß die erfindungsgemäße mehrlagige Tiefziehfolie über eine höhere Dicke als die bekannten Tiefziehfolien verfügt, weist sie überraschenderweise eine verbesserte Knickbruchfestigkeit auf. Diese Verbesserung ist auf den ganz bestimmten, durch die Merkmale des Hauptanspruches gekennzeichneten Aufbau der erfindungsgemäßen Folie zurückzuführen. Einerseits verhindern die Innenschichten aus Äthylen-Vinylacetatcopolymeren infolge ihrer hohen Elastizität eine Knickbildung beim Anlegen eines Vakuums. Andererseits verhärten sie im Gegensatz zu den aus Polypropylen, Polypropylencopolymeren oder aus High-Density-Polyäthylen bestehenden Außenschichten bei einer auf eine Temperaturbelastung folgenden Abkühlung nicht, so daß Knickbrüche infolge Versprödung allenfalls in den Außenschichten auftreten, während die unversehrten Innenschichten auch weiterhin einen sicheren Abschluß des Verpackungsgutes garantieren. Ein weiterer Grund für die verbesserte Knickbruchfestigkeit ist darin zu sehen, daß die Innenlagen miteinander nur durch Verblockung verbunden sind, während sie mit den Außenlagen flächig durch eine Schmelzverbindung verbunden sind, die während der Koextrusion des Schlauches entsteht. Durch die Maßnahme, die beiden Innenlagen lediglich mitein-

- 6 -

ander zu verblocken, wird eine gewisse Beweglichkeit der beiden Innenlagen gegeneinander gewährleistet, die insbesondere beim Tiefziehvorgang und bei der anschließenden Evakuierung von Vorteil ist und ebenfalls zur Verbesserung der Knickbruchfestigkeit beiträgt.

Unter Verblockung wird im Sinne der vorliegenden Erfindung eine Verbindung zwischen den Innenlagen verstanden, die eine mechanische Lösbarkeit dieser beiden Lagen noch ermöglicht. Vorzugsweise ist die durch das Verblocken der Innenlagen erzielte Verbundhaftung nicht größer als 100cN/cm. Die Verblockung erfolgt zweckmäßigerweise unter Anwendung von Wärme und Druck mittels eines Quetschwalzenpaares.

Insbesondere, wenn die Außenlagen der erfindungsgemäßen Folie aus Polypropylen oder einem Polypropylencopolymeren bestehen, ist die Sterilisierbarkeit im Temperaturbereich zwischen 121 $^{\circ}$ und 135 $^{\circ}$C in hervorragender Weise gegeben, und die erfindungsgemäße Folie daher insbesondere für die Verpackung von in Kunststoffbeuteln abgefüllten Blutinfusionsflüssigkeiten geeignet. Aber auch bei der Verwendung von High-Density-Polyäthylen (HDPE, Niederdruckpolyäthylen) als Außenlagenmaterial ist die Verwendung der erfindungsgemäßen Folie für den vorgenannten Einsatzzweck ohne weiteres möglich, wobei in diesem Fall die Sterilisiertemperaturen am unteren Grenzwert von 121 $^{\circ}$C liegen sollten.

Zur Erzielung eines optimalen Tiefziehverhaltens versteht es sich, daß die erfindungsgemäße, durch Koextrusion erhaltene vierlagige Folie im ungereckten Zustand belassen wird.

Ein besonderer Vorteil der erfindungsgemäßen Tiefziehfolie liegt in ihrer ausgezeichneten Verformbarkeit, auch bei der Herstellung von komplizierten Tiefziehformen. Bei der Verpackung von Blutinfusionsflüssigkeiten enthaltenden Kunststoffbeuteln mit Abzapfventil zeigte die erfindungsgemäße Folie, daß mit ihr eine genaue Ausformung der dem Kunststoffbeutel bzw. dem Abzapfventil entsprechenden Tiefzieform möglich ist.

Eine bevorzugte Ausführungsform der Erfindung sieht Außenschichten aus einem Polypropylen-Copolymerisat mit einem Anteil von 1 bis 5 Mol-%, ganz besonders bevorzugt 2 bis 4 Mol-% Äthylen vor. Diese Kunststoffe haben einen Schmelzindex von 0,3 - 15, vorzugsweise von 1 - 10 g/10 Min. bei 230 °C/2,16 kg Last entsprechend ISO/R 133, Prozedur 12.

Diese Propylen-Äthylencopolymeren werden als sogenannte Random-Copolymere bezeichnet. Sie bieten gegenüber Polypropylen-Homopolymerisat den Vorteil, bei niedrigeren Temperaturen versiegelbar zu sein und einen breiteren Siegeltemperaturbereich zu haben. Außerdem verfügen sie über eine höhere Kältefestigkeit.

Zur Erzielung einer hohen Verbundfestigkeit mittels Schmelzverbindung zwischen den Außen- und Innenschichten der erfindungsgemäßen koextrudierten Verbundfolie werden Innenschichten aus einem Äthylen-Vinylacetatcopolymeren bevorzugt, die 10 bis 35 Gew.%, ganz besonders bevorzugt 20 bis 28 Gew.%

Vinylacetat enthalten. Der Schmelzindex dieser Kunststoffe liegt bei 0,5 - 8, vorzugsweise bei 1,5 - 4 g/10 Min. bei 190 °C und 2,16 kg Last gemäß DIN 53735. Diese Innenschichten bieten den weiteren Vorteil, außerordentlich stark miteinander zu verblocken, so daß beim Verblockungsvorgang Lufteinschlüsse vermieden werden. Dadurch können sich bei der zum Sterilisieren notwendigen Erhitzung keine den optischen Eindruck störenden, sichtbaren Blasen ausbilden. Während einer Erhitzung, z.B. beim Sterilisieren des Verpackungsgutes, kann es in Abhängigkeit von der angewendeten Temperatur auch zur Verschmelzung der Innenschichten kommen. Die gute Knickbruchfestigkeit der erfindungsgemäßen koextrudierten Verbundfolie wird aber deswegen nicht beeinträchtigt, weil dieser Schmelzverbund der Innenschichten erst nach den kritischen Verfahrensschritten des Tiefziehens und Evakuierens zustande kommt.

Eine weitere, besonders bevorzugte Ausführungsform sieht vor, Außenschichten eines Copolymeren aus Polypropylen mit 2 bis 4 Mol-% Äthylenanteil mit Innenschichten eines Copolymeren aus Äthylen-Vinylacetat mit einem Vinylacetatanteil von 20 bis 28 Gew.% zu kombinieren. Eine solche vierlagige, koextrudierte Verbundfolie zeigt bei einer Temperatur von 125 °C ein ausgesprochen geschmeidiges Tiefziehverhalten und die Tiefziehmulde erwies sich als besonders zäh und knickbruchfest.

Besonders bevorzugte Ausführungsformen der koextrudierten Verbundfolie sehen eine Gesamtdicke vor, die zwischen 100 und 300 µm liegt, wobei auf die Außenschichten eine Dicke von nicht mehr als 4/5 und nicht weniger als 1/2 und auf die Innenlagen eine Dicke von nicht mehr als 1/2 und nicht weniger als 1/5 entfällt. Die zur Herstellung der Außenlagen gemäß der vorliegenden Erfindung vorgeschlagenen Kunststoffe erfüllen in jedem Fall die Forderung einer guten Verschweißbarkeit mit der Oberfolie einer Tiefziehpackung.

Zur weiteren Erklärung wird die Erfindung anhand der Figuren 1 bis 3 näher erläutert, ohne daß die Erfindung auf die in den Figuren gezeigten Ausführungsformen beschränkt ist.

Figur 1 zeigt in schematischer Darstellung einen im Querschnitt dargestellten zweilagigen koextrudierten Blasschlauch.

Figur 2 zeigt im Querschnitt den gleichen Schlauch, jedoch im verblockten Zustand.

Figur 3 zeigt in einer Schnittdarstellung einen Weich-PVC-Beutel mit Blutinfusionsflüssigkeit, der unter Verwendung der erfindungsgemäßen koextrudierten Verbundfolie als Unterfolie nach dem Vakuumtiefziehverfahren verpackt ist.

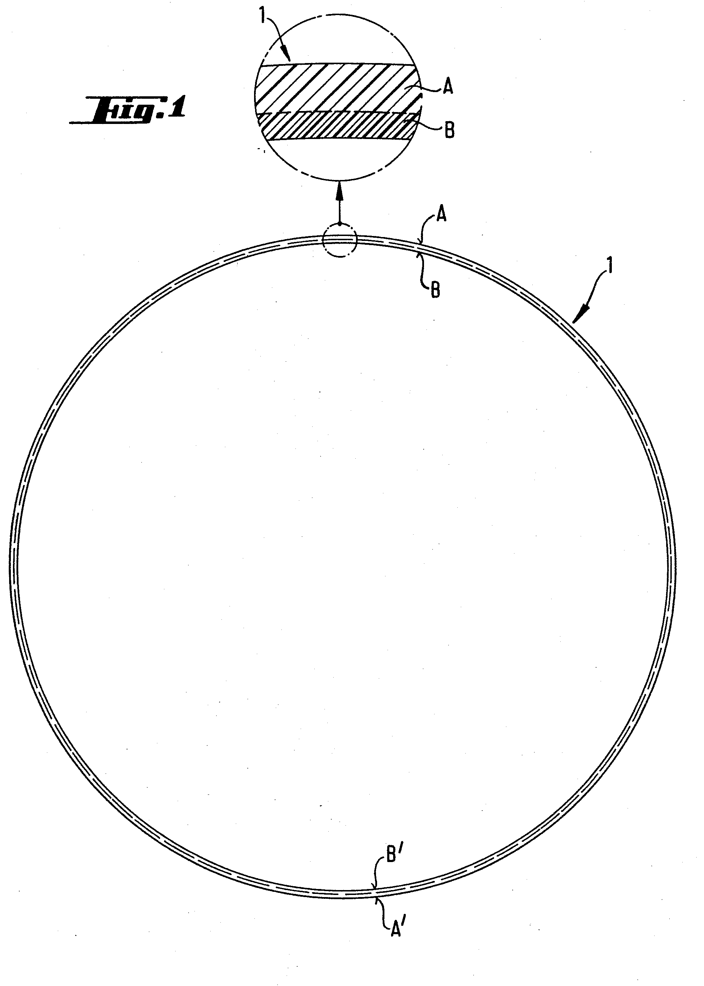

In Figur 1 sind mit A und B die äußere bzw. innere Schicht eines koextrudierten Blasschlauches 1 bezeichnet. Die äußere Schicht A besteht aus einem Copolymeren aus Polypropylen mit 2,7 Mol-% Äthylenanteil und einem Schmelzindex von 3 g/10 Min. Die innere Schicht B besteht aus einem Äthylen-Vinylacetatcopolymeren mit 25 Gew.% Vinylacetatanteil. Schichten A und B sind durch Schmelzbindung miteinander verbunden.

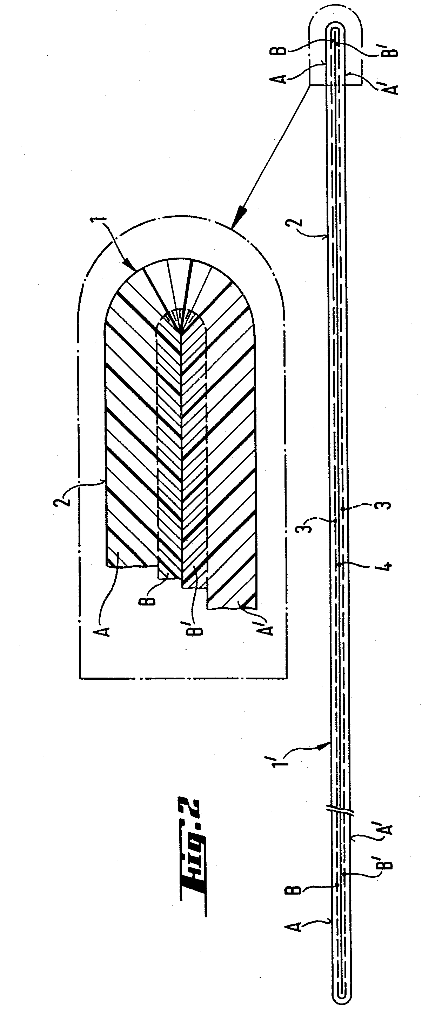

Figur 2 zeigt die durch Flachlegen und Verblocken der Innenschicht B aus dem in Figur 1 dargestellten Blasschlauch 1 gebildete, koextrudierte vierlagige Verbundfolie 1' (verblockter Schlauch) mit ihren aus dem Polypropylencopolymeren gebildeten Außenschichten A, A' und den aus dem Äthylen-Vinylacetatcopolymeren gebildeten Innenschichten B, B'. Die Außenschichten A, A' haben eine Dicke von 70 μm, die Innenschichten B, B' sind 35 μm dick, woraus sich eine Gesamtdicke von 210 μm ergibt.

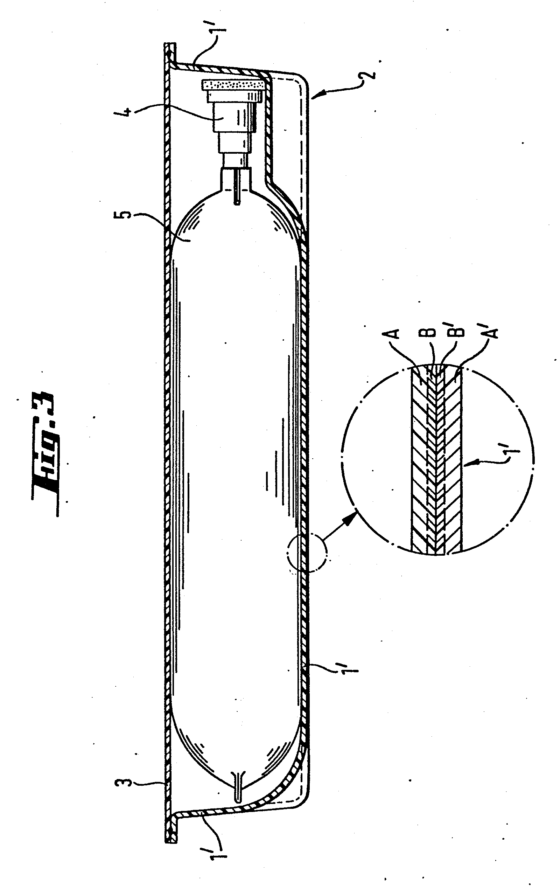

Figur 3 zeigt eine Tiefziehpackung 2, in der ein Blutinfusionsflüssigkeit enthaltender aus Weich-PVC hergestellter Beutel 5 mit Zapfventil 4 verpackt ist. Die Oberfolie 3 ist eine zweilagige Verbundfolie, bestehend aus einer 15 μm dicken, biaxial gereckten Polyamidfolie, laminiert mit einer 50 μm dicken ungereckten Polypropylenfolie. Die Unterfolie ist aus der in Figuren 1 und 2 beschriebenen erfindungsgemäßen koextrudierten Verbundfolie 1' (verblockter Schlauch) gebildet.

0123973

- 11 -

Patentansprüche

1. Mehrlagige sterilisierbare Tiefziehfolie, insbesondere für die Herstellung vakuumtiefgezogener Verpackungen zur Aufnahme von in Kunststoffbeuteln abgefüllten Blutinfusionsflüssigkeiten, gekennzeichnet durch die folgenden Merkmale:

a) die mehrlagige Tiefziehfolie ist eine koextrudierte Verbundfolie (1') mit zwei in Material und Dicke übereinstimmenden Außenlagen (A, A') aus Polypropylen, einem Copolymeren des Polypropylens oder aus High-Density-Polyäthylen (Niederdruckpolyäthylen, HDPE) und zwei in Material und Dicke übereinstimmenden Innenlagen (B, B'), bestehend aus einem Äthylen-Vinylacetatcopolymeren,

b) die Außenlagen (A, A') sind mit den Innenlagen (B, B') durch Schmelzverbindung verbunden, die Innenlagen (B, B') sind lediglich miteinander verblockt.

2. Mehrlagige, sterilisierbare Tiefziehfolie nach Anspruch 1, dadurch gekennzeichnet, daß die Außenschichten (A,A') aus einem Polypropylen-Copolymerisat, mit einem Anteil von 1 bis 5 Mol-%, bevorzugt 2 bis 4 Mol-% Äthylen, bestehen.

3. Mehrlagige, sterilisierbare Tiefziehfolie nach einem der Ansprüche 1 und 2, dadurch gekennzeichnet, daß die Innenschichten (B, B') aus einem Äthylen-Vinylacetatcopolymeren bestehen, das 10 bis 35 Gew.%, bevorzugt 20 bis 28 Gew.% Vinylacetat enthält.

4. Mehrlagige, sterilisierbare Tiefziehfolie nach einem der Ansprüche 1 bis 3, dadurch gekennzeichnet, daß die koextrudierte Verbundfolie (1') eine Gesamtdicke zwischen 60 und 500 µm aufweist, wobei auf die Außenschichten (A, A') eine Dicke von nicht mehr als 4/5 und nicht weniger als 1/2 und auf die Innenlagen (B, B') eine Dicke von nicht mehr als 1/2 und nicht weniger als 1/5 entfällt.

5. Sterilisierbare Vakuumtiefziehverpackung mit darin verpacktem, Blutinfusionsflüssigkeit enthaltenden Kunststoffbeutel aus einer tiefziehfähigen Unterfolie, und einer an sich bekannten, die Packung verschließenden Oberfolie, dadurch gekennzeichnet, daß die Unterfolie der Vakuumtiefziehverpackung (2) eine koextrudierte Verbundfolie (1') mit zwei in Material und Dicke übereinstimmenden Außenlagen (A, A') aus Polypropylen, einem Copolymeren des Polypropylens oder aus High-Density-Polyäthylen (Niederdruckpolyäthylen, HDPE) ist, die durch Schmelzverbindung mit zwei in Material und Dicke übereinstimmenden Innenlagen (B, B') eines Äthylen-Vinylacetatcopolymeren verbunden sind, die ihrerseits miteinander verblockt sind.

0123973

**Fig.1**

0123973

Fig.2

# Fig.3

Europäisches
Patentamt

**EUROPÄISCHER RECHERCHENBERICHT**

0123973
Nummer der Anmeldung

EP 84 10 3997

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| P,X | EP-A-0 093 423 (FELDMÜHLE AG) * Figuren; Zusammenfassung; Ansprüche 1,4,5,10; Seite 9, Zeilen 12-22; Seite 10, Zeilen 7-19; Seite 12, Zeile 29 - Seite 13, Zeile 13 * | 1-5 | B 32 B 27/32 B 65 D 65/40 |
| | --- | | |
| X | FR-A-2 276 918 (OLE-BENDT RASMUSSEN) * Ansprüche 1,2,6-14; Seite 29, Zeilen 10-38; Seite 30, Zeilen 1-13, 34-38; Seite 32, Zeile 26 - Seite 33, Zeile 33 * | 1-5 | |
| | --- | | |
| A | GB-A-1 214 656 (ZELLSTOFFFABRIK WALDHOF) * Ansprüche 1,2; Seite 2, Zeilen 12-54; Seite 3, Zeilen 12-40, 68-75 * | 1 | |
| | --- | | RECHERCHIERTE SACHGEBIETE (Int. Cl. ³) |
| A | DE-A-2 031 036 (OTTO NIELSEN EMBALLAGE A/S) * Figuren 6a,6b; Ansprüche 1-5; Seite 2, Zeilen 12-23; Seite 4, Zeile 11 - Seite 6, Zeile 10; Seite 11, Zeilen 3-5 * | 1 | B 32 B |
| | --- | | |
| A | GB-A-1 318 745 (DOW CHEMICAL COMPANY) * Figur 1A; Seite 3, Zeile 74 - Seite 4, Zeile 28 * | 1,3 | |
| | ----- | | |

Der vorliegende Recherchenbericht wurde für alle Patentansprüche erstellt.

| Recherchenort DEN HAAG | Abschlußdatum der Recherche 06-08-1984 | Prüfer DE LA MORINERIE B.M. |
|---|---|---|

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82